# EUROPÄISCHE PATENTSCHRIFT

(11) **EP 0 614 649 B1**
(45) Veröffentlichungstag und Bekanntmachung des Hinweises auf die Patenterteilung: **08.05.1996**
(21) Anmeldenummer: 94102050.5
(22) Anmeldetag: 10.02.1994
(51) Int. Cl.: A61B 17/60

(54) **Knochenschraube**
Bone screw
Vis à os

(30) Priorität: 10.03.1993 DE 4307576
(43) Veröffentlichungstag der Anmeldung: 14.09.1994
(73) Patentinhaber: BIEDERMANN MOTECH GmbH, D-78054 Villingen-Schwenningen (DE)
(72) Erfinder: Biedermann, Lutz, D-78054 VS-Schwenningen (DE); Harms, Jürgen, Prof. Dr., D-76337 Waldbronn-Reichenbach (DE)
(74) Vertreter: Prüfer, Lutz H., Dipl.-Phys.

(56) Entgegenhaltungen:
- WO-A-91/01115
- DE-U- 9 202 745

## Beschreibung

Die Erfindung betrifft eine Knochenschraube mit einem Schraubenelement und einem Aufnahmeteil für die Aufnahme des Schraubenelementes und einer mit dem Schraubenelement zu verbindenden Stange.

Aus DE-U-92 02 745 ist eine derartige Knochenschraube bekannt. Diese weist ein einen Gewindeabschnitt und einen kugelförmigen Abschnitt besitzenden Kopf aufweisendes Schraubenelement und ein Aufnahmeteil für die Aufnahme des Kopfes des Schraubenelementes und einer mit der Knochenschraube zu verbindenden Stange auf. Das Aufnahmeteil besitzt an seinem einen Ende eine erste Bohrung zum Hindurchführen des Gewindeabschnittes und angrenzend an diese innen einen hohlkugelförmigen Abschnitt zum Anlegen des Kopfes. Unmittelbar an den hohlkugelförmigen Abschnitt schließt sich auf der der ersten Bohrung gegenüberliegenden Seite eine kurze zylindrische Bohrung zum Einführen des Gewindeschaftteiles mit Kopf an. An dem der ersten Bohrung abgewandten freien Rand des Abschnittes der zylindrischen Bohrung schließen sich zwei zueinander parallele ebene Schenkel an, die dazu dienen, die mit dem Schraubenelement zu verbindende Stange zwischen sich aufzunehmen. An ihrem freien Ende weisen die beiden Schenkel Gewindeabschnitte zur Aufnahme einer Sicherungsschraube auf.

Aus der EP-0 465 158 A ist ein Verankerungselement mit einem zur Knochenverankerung bestimmten Schaft und einem mit einer Stange verbindbaren Kopf mit einem im wesentlichen U-förmigen Querschnitt, der an seinem Grund mit dem Schaft verbunden ist und zwei einen Kanal zur Aufnahme der Stange bildende freie Schenkel hat, wobei die freien Schenkel ein sich in Richtung der Schenkel erstreckendes erstes Innengwinde aufweisen, und ein Fixierelement und ein die Schenkel von außen umfassendes Element vorgesehen sind, bekannt. Das Fixierelement und das die Schenkel von außen umfassende Element sind über einen Zwischeneinsatz miteinander verbunden, der seinerseits in das Innengewinde der beiden freien Schenkel eingeschraubt ist. Bei dieser bekannten Verankerung ist es erforderlich, daß stets zunächst das Zwischenstück mit dem umfassenden Element fixiert und erst dann das Fixierelement innen eingeschraubt wird. Aus der EP-0 443 892 A ist eine Vorrichtung bekannt, bei der um freie Schenkel eines Kopfes ein Außenring gesetzt wird, der eine in den Kanal greifende Zunge aufweist. Es ist ein in ein Innengewinde einschraubbares Fixierelement vorgesehen, welches bei Fixierung auf die Zunge einwirkt. Eine getrennte Bewegung von dem umfassenden Element und dem Fixierelement zum Handhaben der Vorrichtung ist nicht vorgesehen. Eine Schraubensicherung ist ebenfalls nicht vorgesehen. Die Fixierung eines gebogenen Stabes ist nicht möglich.

Aufgabe der Erfindung ist es, eine Knochenschraube zu schaffen, die so ausgebildet ist, daß sie insbesondere bei der Behandlung von Wirbelsäulenkorrekturen bei der Fixation eines Rundstabes universell einsetzbar ist, insbesondere auch dann, wenn die aufzunehmende Stange gebogen ist.

Diese Aufgabe wird mit einer Knochenschraube mit den im Patentanspruch 1 angegebenen Markmalen gelöst.

Weiterbildungen der Erfindung sind in den Unteransprüchen gekennzeichnet.

Weitere Merkmale und Zweckmäßigkeiten ergeben sich aus der Beschreibung von Ausführungsbeispielen anhand der Figuren.

Von den Figuren zeigen:
- Fig. 1: eine Explosionsdarstellung einer ersten Ausführungsform, teilweise in geschnittener Darstellung;
- Fig. 2: die in Fig. 1 gezeigte Vorrichtung in zusammengesetztem Zustand, in vergrößertem Maßstab;
- Fig. 3: eine Explosionsdarstellung einer anderen Ausführungsform; und
- Fig. 4: eine Darstellung der in Fig. 3 gezeigten Vorrichtung in zusammengesetztem Zustand und größerem Maßstab.

Die Knochenschraube weist das eigentliche Schraubenelement 1 mit einem Gewindeabschnitt 2 und einem Kopf 3 auf. Der Kopf 3 ist angrenzend an den Gewindeabschnitt kugelsegmentförmig ausgebildet. Koaxial zur Gewindeachse und auf dem dem Gewindeabschnitt 2 gegenüberliegenden Ende weist der Kopf eine Ausnehmung 4 zum Ineingriffbringen mit einem Imbusschlüssel auf.

Die Knochenschraube umfaßt ferner ein zylindrisch ausgebildetes Aufnahmeteil 5. Dieses weist an seinem einen Ende eine axialsymmetrisch ausgerichtete erste Bohrung 7 auf, deren Durchmesser größer als der des Gewindeabschnittes 2 und kleiner als der des Kopfes 3 ist. Das Aufnahmeteil 5 weist ferner eine koaxiale zweite Bohrung 8 auf, die auf dem der ersten Bohrung 7 gegenüberliegenden Ende offen ist und deren Durchmesser so groß ist, daß das Schraubenelement durch das offene Ende mit seinem Gewindeabschnitt durch die erste Bohrung 7 hindurch und mit dem Kopf 3 bis zum Grund der zweiten Bohrung führbar ist. Zwischen der ersten und der zweiten Bohrung ist ein kleiner koaxialer Abschnitt 9 vorgesehen, der unmittelbar an die erste Bohrung angrenzt und zum offenen Ende hin sphärisch ausgebildet ist, wobei der Radius im wesentlichen gleich dem Radius des kugelsegmentförmigen Abschnittes des Kopfes 3 ist. Angrenzend an den Abschnitt 9 weist die zweite Bohrung ein sich koaxial bis zum freien Ende hin erstreckendes Innengeweinde 10 auf. Ferner weist das Aufnahmeteil 5 eine zur Mitte des Teiles symmetrisch angeordnete U-förmige Ausnehmung 6 auf, deren Grund zu der ersten Bohrung 7 hin gerichtet ist und deren beide Seitenschenkel sich zu dem der ersten Bohrung abgewandten offenen Ende hin erstrecken. Am freien Ende der Schenkel der U-förmigen Ausnehmung ist ein Außengewinde 11 vorgesehen.

Das Schraubenelement umfaßt ferner eine Kopf-Fixierschraube 12 und eine Stab-Fixierschraube 13, die beide ein mit dem Innengewinde 10 zusammenpassendes Außengewinde aufweisen. Die Kopf-Fixierschraube weist auf ihrer dem Kopf 3 zugewandten Seite eine sphärische Ansenkung 15 auf. Beide Fixierschrauben 12, 13 weisen jeweils Sechskant-Innenflächen zum Ineingriffbringen mit einem Imbus-Schraubendreher auf. Darüber hinaus ist eine Sicherungsmutter 14 vorgesehen mit einem Innengewinde, welches mit dem Außengewinde 11 zusammenpaßt.

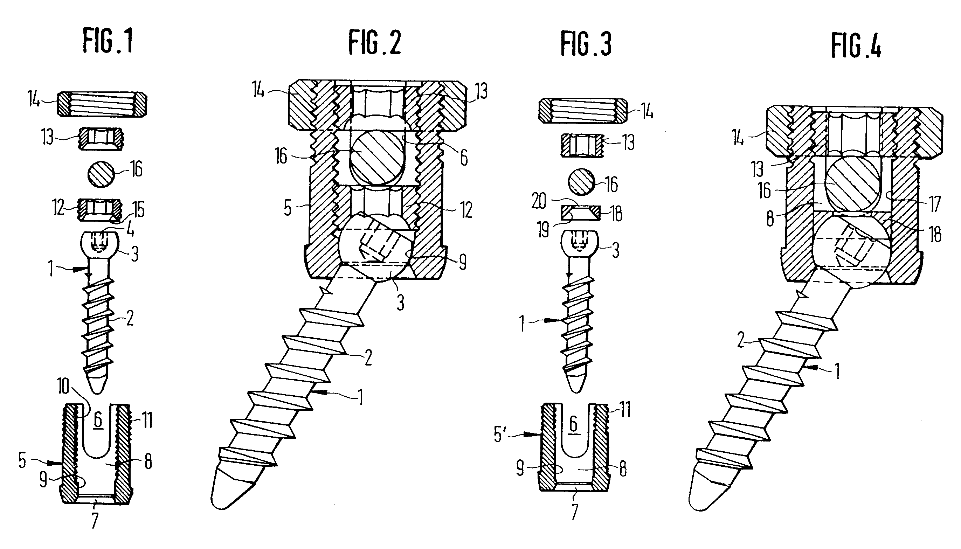

Im Betrieb wird das Schraubenelement 1 von dem offenen Ende der zweiten Bohrung her in die erste Bohrung hineingeführt. Durch Eingreifen an der Ausnehmung 4 wird das Schraubenelement 1 in die Wirbelsäule eingeschraubt. Anschließend wird die Kopf-Fixierschraube mit einem Imbus-Schraubendreher zunächst lose bis zum Kopf 3 hin eingeschraubt. Es erfolgt dann eine korrekte Ausrichtung des Aufnahmeteiles 5 zur Aufnahme eines mit der Knochenschraube zu verbindenden Rundstabes 16. In dieser ausgerichteten Stellung wird die Kopf-Fixierschraube so fest angezogen, daß Schraubenelement 1 und Aufnahmeteil 5 eine feste Verbindung besitzen. Anschließende wird der Rundstab 16 in die U-förmige Ausnehmung 6 endgültig eingelegt und durch Einsetzen und Festziehen der Stab-Fixierschraube 13 in seiner Position fixiert. Dann wird die Sicherungsmutter 14 in der in Fig. 2 gezeigten Weise auf die freien Enden der Schenkel der U-förmigen Ausnehmung 6 aufgeschraubt. Sowohl die Stab-Fixierschraube 13 als auch die Sicherungsmutter 14 werden so weit gedreht, bis sie eine gewünschte Haltekraft auf den Rundstab 16 ausüben. Dadurch wird der Vorteil erreicht, daß die auf den Stab 16 einwirkende Kraft von der Stab-Fixierschraube 13 bzw. der Sicherungsmutter 14 unabhängig voneinander einstellbar ist. Darüber hinaus erfolgt eine endgültige Fixation und eine Schraubensicherung durch Verklemmung.

Die getrennte Fixierung durch die Stab-Fixierschraube 13 und die Sicherungsmutter 14 ist besonders deshalb wichtig, weil es nicht nur gerade Stäbe, sondern auch gekrümmte Stäbe zu fixieren gilt. Hier ist eine exakte Fixierung nur dadurch möglich, daß die beiden Elemente getrennt voneinander einstellbar sind.

Wie am besten aus Fig. 2 ersichtlich ist, ist die erste Bohrung 7 so konisch ausgebildet, daß sie nach außen hin einen größeren Durchmesser als in dem an den Abschnitt 9 angrenzenden Teil aufweist. Dadurch ist es möglich, das Schraubenelement 1 in der in Fig. 2 gezeigten Weise um einen Winkel zur Symmetrieachse des Aufnahmeteiles 5 zu schwenken.

Im folgenden wird die in den Figuren 3 und 4 gezeigte zweite Ausführungsform beschrieben. Übereinstimmende Teile sind jeweils mit den gleichen Bezugszeichen versehen.

Das Schraubenelement 1, die Stab-Fixierschraube 13 und die Sicherungsmutter 14 stimmen in allen Einzelheiten mit der ersten Ausführungsform überein. Abweichend von dem oben beschriebenen Ausführungsbeispiel ist zwischen dem Abschnitt 9 und dem Innengewinde 10 ein Zylinderabschnitt 17 ohne Innengewinde vorgesehen. Anstelle der Kopf-Fixierschraube ist eine Druckscheibe 18 vorgesehen, die so ausgebildet ist, daß sie auf ihrer dem Kopf 3 zugewandten Seite eine sphärische Ansenkung 19 aufweist, deren Radius im wesentlichen gleich dem Radius des kugelsegmentförmigen Abschnittes des Kopfes 3 ist. Der Außendurchmesser der Druckscheibe 18 ist so gewählt, daß die Druckscheibe 18 in dem Zylinderabschnitt 17 eine Gleitbewegung ausführen kann, also in dem Zylinderabschnitt 17 zu dem Kopf hin verschiebbar ist. In allen übrigen Elementen stimmen die beiden Ausführungsformen überein. Vorzugsweise weist die Druckscheibe 18 eine koaxiale Bohrung 20 auf, die einen Zugriff zur Ausnehmung 4 und auch ein leichteres Zugreifen auf die Druckscheibe 18 ermöglicht.

Im Betrieb wird wie bei der ersten Ausführungsform zunächst das Schraubenelement 1 nach Einsetzen in das Aufnahmeteil 5 eingeschraubt. Dann werden nacheinander die Druckscheibe 18 und der Stab 16 eingesetzt. Anders als beim ersten Ausführungsbeispiel sind in diesem Stadium das Schraubenelement 1 und das Aufnahmeteil 5 noch voll gegeneinander schwenkbar. Erst durch Einschrauben der Stab-Fixierschraube 13 werden Schraubenelement 1 und Aufnahmeteil 5 zueinander und damit auch der Stab 16 fixiert. Dann wird wiederum die Sicherungsmutter 14 aufgeschraubt. Sicherungsmutter 14 und Stabfixierschraube 13 werden getrennt wieder so weit in Richtung zum Grund der Bohrung gedreht, bis jedes der beiden Teile eine gewünschte Haltekraft auf den Stab 16 ausübt.

Mit den beiden oben beschriebenen Ausführungsformen wird einmal eine Verbindung geschaffen, bei der das Schraubenelement und Aufnahmeteil vor der Fixierung des Stabes 16 schon eine feste Verbindung und damit eine feste Stellung zueinander einnehmen, während im zweiten Ausführungsbeispiel die endgültige Ausrichtung erst durch Festziehen der Stab-Fixierschraube 13 erfolgt.

In einer dritten Ausführungsform stimmen alle Teile mit der ersten Ausführungsform überein. Zusätzlich oder alternativ zu der Kopf-Fixierschraube 12 ist eine Druckscheibe entsprechend der Druckscheibe 18 vorgesehen, deren Außendurchmesser so gewählt ist, daß sie entlang des Innengewindes im gleichen Sinne wie bei der zweiten Ausführungsform gleitend zu dem Kopf 3 hin verschiebbar ist. Die Fixierung erfolgt bei Verwendung der Druckscheibe dann in der gleichen Weise wie bei der zweiten Ausführungsform.

Die beschriebene dritte Ausführungsform hat den Vorteil, daß die Lagerhalterung vermindert werden kann, da nur eine Art von Aufnahmeteil 5 erforderlich ist, die beide Anwendungsmöglichkeiten gestattet.

## Patentansprüche

1. Knochenschraube mit einem einen Gewindeabschnitt (2) und einen kugelsegmentförmigen Abschnitt besitzenden Kopf (3) aufweisenden Schraubenelement (1)
und einem zylindrischen Aufnahmeteil (5) für die Aufnahme des Kopfes (3) des Schraubenelementes (1) und einer mit der Knochenschraube zu verbindenden Stange (16),
wobei das Aufnahmeteil (5) an seinem einen Ende eine erste Bohrung (7) zum Hindurchführen des Gewindeabschnittes (2), angrenzend an diese innen einen hohlkugelförmigen Abschnitt (9) zum Anlegen des Kopfes (3), eine auf der der ersten Bohrung (7) gegenüberliegenden Seite offene zweite Bohrung (8) zum Einführen des Gewindeschaftteiles (2) mit Kopf (3), einen im wesentlichen U-förmigen Querschnitt mit zwei freien, ein Außengewinde besitzenden Schenkein und ein Innengewinde (10) aufweist, und wobei
ein auf den Kopf (3) einwirkendes Druckelement (12, 18), eine an der offenen Seite über der in den U-förmigen Querschnitt einzusetzenden Stange (16) eingeschraubte Klemmschraube (13) und eine auf dem Außengewinde (11) aufgeschraubte Sicherungsmutter (14) vorgesehen sind.

2. Knochenschraube nach Anspruch 1 dadurch gekennzeichnet, daß der Kopf (3) auf seiner dem Gewindeabschnitt (2) abgewandten Seite einen mit einem Schraubendreher in Eingriff bringbaren Abschnitt (4) aufweist.

3. Knochenschraube nach Anspruch 1 oder 2, dadurch gekennzeichnet, daß
das Druckelement (12, 18) auf seiner dem Kopf (3) zugewandten Seite einen zu dem Kopf hin gerichteten hohlkugelsegmentförmigen Abschnitt (15, 19) aufweist.

4. Knochenschraube nach einem der Ansprüche 1 bis 3, dadurch gekennzeichnet, daß
das Druckelement (18) als Scheibe ausgebildet ist.

5. Knochenschraube nach einem der Ansprüche 1 bis 3, dadurch gekennzeichnet, daß
das Druckelement (13) als Schraube ausgebildet ist.

## Claims

1. A bone screw with a screw element (1) having a threaded section (2) and a head having a spherical segment-shaped section,
and a cylindrical receiving portion (5) for receiving the head (3) of the screw element (1) and a rod (16) to be connected to the bone screw,
in which arrangement the receiving portion (5) has at one of its ends a first bore (7) for passing the threaded section (2) through, adjoining it on the inside a hollow spherical section (9) for the positioning of the head (3), an open second bore (8) lying on the side opposite the first bore (7) for introducing the threaded section (2) with the head (3), a substantially U-shaped cross-section with two free sides having an outer thread and an inner thread (10), and in which arrangement there are provided,
a pressure element (12, 18) acting on the head (3), a clamping screw (13) screwed in at the open side above the rod (16) to be inserted into the U-shaped cross-section, and a lock nut (14) screwed onto the outer thread (11).

2. A bone screw according to claim 1, characterized in that the head (3) has, on its side remote from the threaded section (2), a section (4) that can be brought into engagement with a screwdriver.

3. A bone screw according to claim 1 or 2, characterized in that
the pressure element (12, 18) has, on its side remote from the head (3), a hollow spherical segment-shaped section (15, 19) directed towards the head.

4. A bone screw according to one of claims 1 to 3, characterized in that
the pressure element (18) is designed as a washer.

5. A bone screw according to one of claims 1 to 3, characterized in that
the pressure element (13) is designed as a screw.

## Revendications

1. Vis à os comprenant un élément vis (1) présentant une partie filetée (2) et une tête (3) qui possède une partie en forme de segment sphérique,
et une pièce réceptrice cylindrique (5) destinée à recevoir la tête (3) de l'élément vis (1) et une barre (16) qui doit être assemblée à la vis à os,
dans laquelle la pièce réceptrice (5) présente, à une de ses extrémités, un premier perçage (7) destiné à laisser passer la partie filetée (2), à la suite de ce perçage vers l'intérieur, une partie (9) de forme sphérique creuse destinée à l'application de la tête (3), un deuxième perçage (8) qui s'ouvre sur le côté qui est à l'opposé du premier perçage (7), et destiné à l'introduction de la partie (2) à fût fileté munie de la tête (3), une section sensiblement en forme de U possédant deux branches libres qui présentent un filetage extérieur et un filetage intérieur (10), et dans laquelle sont prévus un élément de pression (12, 18) agissant sur la tête (3), une vis de coincement (13) vissée dans le côté ouvert, au-dessus de la barre (16) destinée à être placée dans la section en forme de U, et un écrou de blocage (14) vissé sur le filetage extérieur.

2. Vis à os selon la revendication 1, caractérisée en ce que la tête (3) présente, sur son côté qui est éloigné de la partie filetée (2), une partie (4) qui peut être mise en prise avec un tournevis.

3. Vis à os selon la revendication 1 ou 2, caractérisée en ce que
l'élément de pression (12, 18) présente, sur son côté dirigé vers la tête (3), une partie (15, 19) ayant la forme d'une partie sphérique creuse dirigée vers la tête.

4. Vis à os selon l'une des revendications 1 à 3, caractérisée en ce que
l'élément de pression (18) est constitué par une rondelle.

5. Vis à os selon l'une des revendications 1 à 3, caractérisée en ce que
l'élément de pression (13) est constitué par une vis.
